# EUROPEAN PATENT APPLICATION

(11) **EP 4 674 850 A1**
(43) Date of publication of application: **07.01.2026**
(21) Application number: 24763144.3
(22) Date of filing: 27.02.2024
(51) Int. Cl.: C07D 473/06, C07D 473/08, A61K 31/522, A61P 13/04, A61P 19/06, A61P 29/00, A61P 19/02, A61P 13/12

(54) **CRYSTAL FORM OF 8-CHLORO-3-PENTYL-3,7-DIHYDRO-1H-PURIN-2,6-DIONE COMPOUND AND PREPARATION METHOD THEREFOR**

(30) Priority: 27.02.2023 CN 202310170276
(71) Applicant: Shanton Pharma Pte. Ltd., Singapore 068912 (SG)
(72) Inventor: MATSUYAMA, Koji, Shanghai 200122 (CN); ZHANG, Qian, Shanghai 200122 (CN)
(74) Representative: Patentanwälte Ruff, Wilhelm, Beier, Dauster & Partner mbB
(86) International application number: PCT/CN2024/078799
(87) International publication number: WO 2024/179463

(57) **Abstract**

The present invention belongs to the technical field of medicine; relates to a crystal form of an 8-chloro-3-pentyl-3,7-dihydro-1H-purin-2,6-dione compound and a preparation method therefor; and particularly relates to a crystal form of a compound as represented by formula (1), a method for preparing the crystal form of the compound as represented by formula (1), a composition and a preparation containing the crystal form, and the use of the crystal form thereof or the composition and the preparation containing the crystal form in the preparation of a drug for reducing uric acid and resisting inflammation and/or treating and/or preventing uric acid diseases and/or gouty diseases.

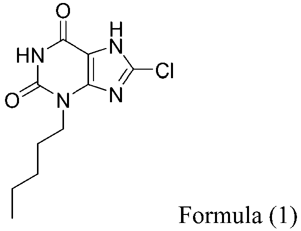

## Description

### Technical field

The present invention belongs to the field of medical technology, and specifically relates to crystal forms of the compound 8-chloro-3-pentyl-3,7-dihydro-1H-purine-2,6-dione, a composition and a formulation containing said crystal form, and a preparation method thereof, as well as use of crystal forms of the compound or the composition or formulation containing said crystal form in manufacture of medicament for lowering uric acid, reducing inflammation and/or treating and/or preventing uratic or gouty diseases.

### Background technology

Hyperuricemia is a metabolic disease caused by disorders in purine metabolism leading to an increase in uric acid in the blood. As uric acid levels in the blood continuously rise, excessively high concentrations of uric acid in the body lead to the appearance of uric acid crystals. These uric acid crystals can trigger inflammatory reactions, resulting in gout attacks. Consequently, some gout patients develop the condition after progressing from asymptomatic hyperuricemia through this process of crystal formation.

Uric acid crystals typically occur in joints, kidneys, and soft tissues, causing persistent and long-term pain for patients, requiring long-term treatment with uric acid-lowering medications. After gout patients start taking uric acid-lowering drugs, fluctuations in blood uric acid levels can cause partial dissolution of tophi or urate crystal surfaces, thereby triggering gout attacks. The greater the degree of reduction in blood uric acid levels, the greater the chance of a gout attack. This process causes significant suffering for patients and may even prevent them from continuing medication. Even using drugs for acute gout attacks concurrently with uric acid-lowering drugs is often insufficient to eliminate the patient's suffering.

Currently, anti-gout drugs mainly include drugs for acute gout attacks (colchicine, non-steroidal anti-inflammatory drugs, glucocorticoids), uric acid synthesis inhibitors (such as allopurinol and febuxostat), and uric acid excretion promoters (such as probenecid and benzbromarone). During acute gout attacks, colchicine, non-steroidal anti-inflammatory drugs, and glucocorticoids are primarily used. Uric acid-lowering therapy mainly uses uric acid synthesis inhibitors and uric acid excretion promoters. These drugs have defects such as poor efficacy and significant side effects.

It is believed that the plasma concentration of uric acid-lowering drugs is related to the degree of reduction in blood uric acid levels, and the degree of reduction in blood uric acid levels is related to the frequency of gout attacks (Dalbeth, Nicola, et al. "2020 American College of Rheumatology Guideline for the Management of Gout." Arthritis Care & Research 72.6 (2020)).To reduce the risk of acute gout attacks during the initial treatment with uric acid-lowering drugs, controlling fluctuations in the plasma concentration of these drugs is crucial. Clinical guidelines and practice recommend a stepwise dosing regimen for uric acid-lowering therapy (e.g., allopurinol, febuxostat, benzbromarone) starting with a low dose and slowly increasing. Furthermore, controlling fluctuations in plasma drug concentration after the uric acid-lowering drug reaches a steady state is even more important for reducing the frequency of gout attacks. Therefore, uric acid-lowering drugs with a longer half-life to reduce the peak-to-trough ratio of plasma drug concentration have significant clinical significance for gout treatment.

WO2016/119570A1 discloses a series of compounds useful for lowering uric acid, reducing inflammation, and preventing or treating uratic or gouty diseases. Among them, the compound 8-chloro-3-pentyl-3,7-dihydro-1H-purine-2,6-dione (Formula (1)) has a good uric acid-lowering effect.

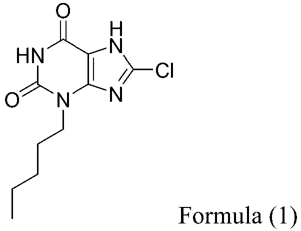

WO2016/119570A1 discloses the preparation and column chromatography separation method of 8-chloro-3-pentyl-3,7-dihydro-1H-purine-2,6-dione, ultimately obtaining a pale yellow solid (Example 2). This reference does not study the crystal forms of the compound.

WO2005077950A2 relates to drugs having HM74A activity. The drugs involved in this reference are not related to lowering uric acid, but it also discloses the preparation of 8-chloro-3-pentyl-3,7-dihydro-1H-purine-2,6-dione, ultimately producing a white solid (Example 12). This reference also does not study the crystal forms of the compound.

To maximize the efficacy of drugs with HM74A activity, it is necessary to achieve an increased Cmax and a decreased Tmax. Therefore, based on WO2005077950A2, aiming to increase Cmax and decrease Tmax, WO2010/068581A1 discloses a crystal form of 8-chloro-3-pentyl-3,7-dihydro-1H-purine-2,6-dione tris(hydroxymethyl)aminomethane salt. This crystal form is used to treat diseases caused by insufficient activation of the HM74A receptor or diseases benefiting from activation of the HM74A receptor. It is more stable and has higher solubility than the free acid, exhibiting increased Cmax and decreased Tmax, facilitating faster absorption by patients.

However, for uric acid-lowering drugs, excessively large peak-to-trough fluctuations in drug concentration are undesirable to avoid adverse reactions such as gout attacks caused by rapid decreases in blood uric acid. Further research shows that the compound 8-chloro-3-pentyl-3,7-dihydro-1H-purine-2,6-dione has a significant uric acid-lowering effect. Therefore, a uric acid-lowering drug with a long half-life can reduce the problem of gout attacks caused by drug concentration fluctuations.

Furthermore, each tris(hydroxymethyl)aminomethane molecule has three hydroxyl groups. Due to the high polarity of hydroxyl groups, it is believed that the 8-chloro-3-pentyl-3,7-dihydro-1H-purine-2,6-dione tris(hydroxymethyl)aminomethane salt has high hygroscopicity, which is a disadvantageous factor for pharmaceutical use. On the other hand, the molecular weight of tri(hydroxymethyl)aminomethane is relatively high, while the molecular weight of the parent compound 8-chloro-3-pentyl-3,7-dihydro-1H-purine-2,6-dione itself is not very high. Therefore, when 8-chloro-3-pentyl-3,7-dihydro-1H-purine-2,6-dione converts to its tris(hydroxymethyl)aminomethane salt, the content of the active ingredient in the drug product is significantly reduced, which is detrimental to patient compliance.

Therefore, there is an urgent need to find stable, pharmaceutically acceptable crystal forms of the compound of formula (1) with a relatively long half-life for use in preparing drugs for lowering uric acid, reducing inflammation, treating and/or preventing uratic and/or gouty diseases.

### Summary of the invention

During the research and development process of the compound of formula (1), it was found that the free acid of the compound of formula (1) can easily transform (or partially transform) into another form through hydration during the main preparation process. To obtain a crystal form with high purity, high content, good stability, and suitability for industrial production, the inventors have conducted extensive research on the crystal forms of the compound of formula (1) and obtained crystal forms of the compound of formula (1).

A first object of the present invention is to provide crystal forms of the compound of formula (1).

A second object of the present invention is to provide pharmaceutically acceptable crystal forms of the compound of formula (1).

A third object of the present invention is to provide methods for preparing crystal forms of the compound of formula (1) and methods for mutual transformation of said crystal forms.

Another object of the present invention is to provide use of the crystal forms of the compound of formula (1) in manufacture of medicament for lowering uric acid, reducing inflammation, and/or treating and/or preventing uratic and/or gouty diseases, wherein the uratic disease includes hyperuricemia, uratic nephropathy and the like, and the gouty disease includes gout, gouty inflammation and the like.

Another object of the present invention is to provide crystal forms of the compound of formula (1) exhibiting an extended half-life, thereby minimizing the problem of gout attacks caused by fluctuations in plasma drug concentration and improving patient compliance.

### Crystal forms of the compound 8-chloro-3-pentyl-3,7-dihydro-1H-purine-2,6-dione represented by formula (1)

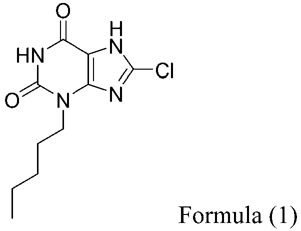

### (1) Crystal forms I, II, III, IV, V, and VI

The present inventors have conducted extensive research on crystal forms of the compound of formula (1) and discovered crystal forms I, II, III, IV, V, and VI of the compound of formula (1). It is specifically pointed out that these six different crystal forms cannot be predicted based on the chemical formula of the compound of formula (1), nor is it possible to predict the structure or properties of any of these crystal forms.

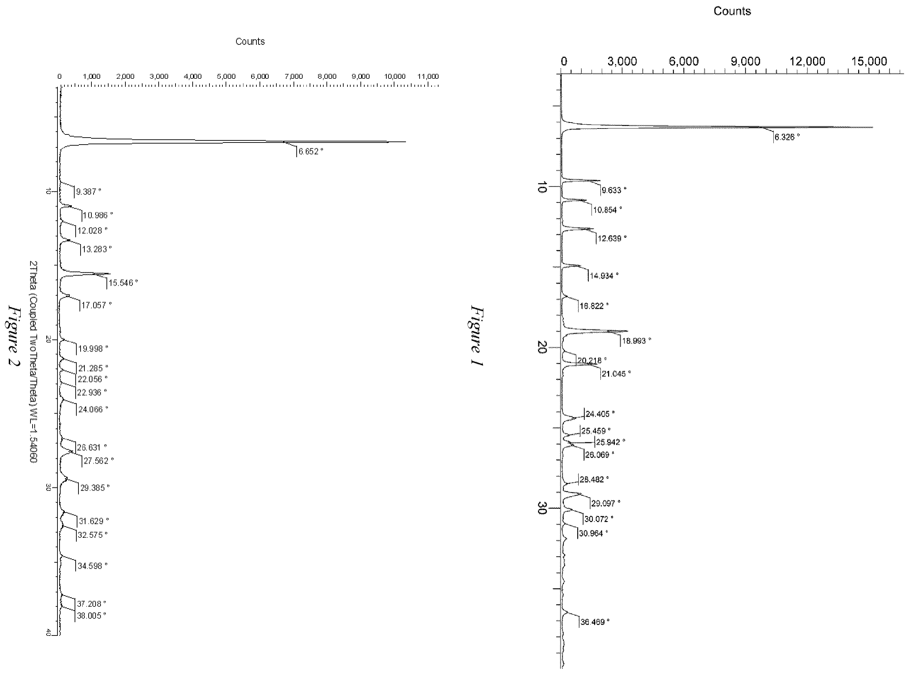

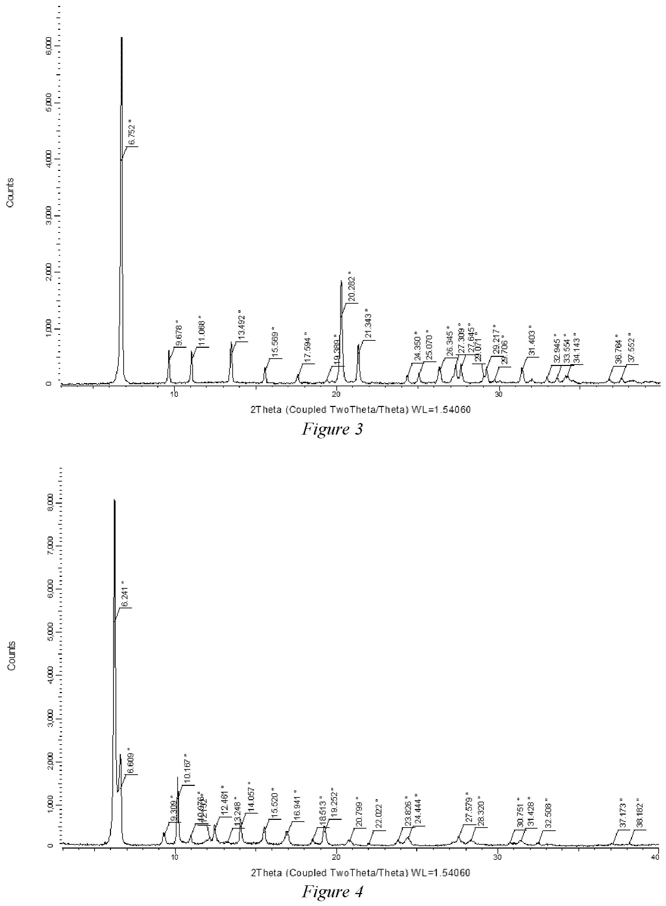

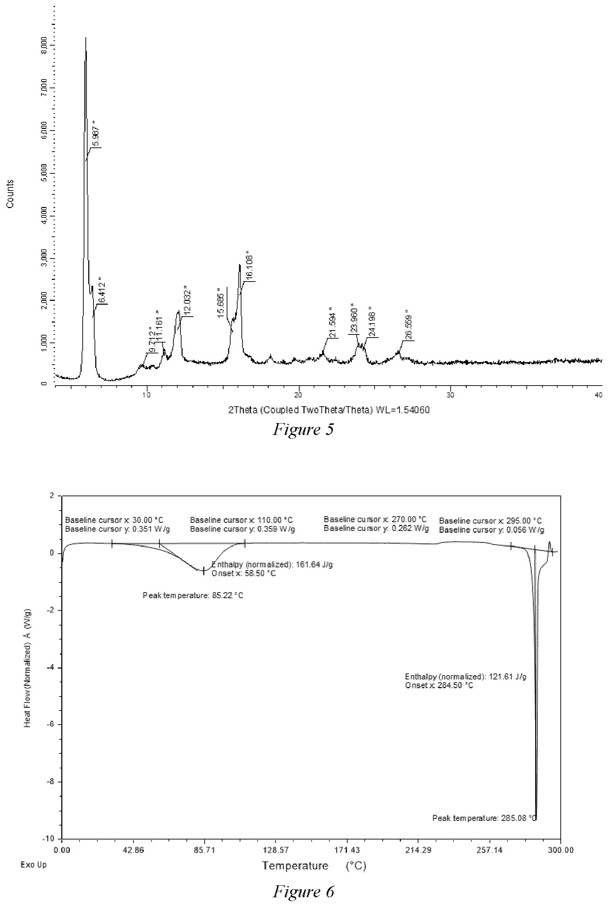

The crystal forms of the compound of formula (1) are characterized in that,
the X-ray powder diffraction pattern of crystal form I is shown in Figure 1;
the X-ray powder diffraction pattern of crystal form II is shown in Figure 2;
the X-ray powder diffraction pattern of crystal form III is shown in Figure 3;
the X-ray powder diffraction pattern of crystal form IV is shown in Figure 4; and
the X-ray powder diffraction pattern of crystal form V is shown in Figure 5.

It is found in the research that crystal form I is a monohydrate and crystal form II is an anhydrate.

Crystal form III is a monohydrate with high crystallinity; when crystal form III is heated to 95°C in a water-alcohol or ketone (e.g., C1-3alcohol or acetone) system and maintained for 10 minutes, then cooled to ambient conditions (20-25°C, 70-80% RH), it transforms into crystal form II.

Crystal form IV is a monohydrate with high crystallinity; it can be obtained by heating crystal form I to 150°C, then cooling to ambient conditions (20-25°C, 70-85% RH), or by placing crystal form V under ambient conditions (25-30°C, 70-85% RH) for about 10 hours.

Crystal form V is an anhydrate; it can be obtained by heating crystal form I to 100°C and maintaining for 30 minutes. Crystal form V has a moderate crystallinity and is a metastable crystal form, which can transform into crystal form IV after being placed under ambient conditions (25-30°C, 70-85% RH) for about 10 hours.

Crystal form VI can be obtained in methanol. It can ultimately transform into the anhydrate crystal form II after drying (via crystal form III).

In summary, not all of crystal forms I, II, III, IV, V and VI of the compound of formula (1) meet pharmaceutical requirements. Among them, crystal forms V and VI are in metastable states and cannot meet clinical needs.

### (2) Pharmaceutically acceptable crystal forms

Based on the above research, the present invention provides stable crystal forms I, II, III and IV of the compound of formula (1).

Preferred technical solutions of the present invention are outlined as follows:
Crystal forms of the compound 8-chloro-3-pentyl-3,7-dihydro-1H-purine-2,6-dione represented by formula (1) have X-ray powder diffraction patterns comprising the following characteristic peaks expressed by 2θ degree, when measured using Cu-Kalpha radiation:
Crystal form I: 6.3°±0.2°, 9.6°±0.2°, 19.0°±0.2°, 21.0°±0.2°;
Crystal form II: 6.7°±0.2°, 11.0°±0.2°, 15.5°±0.2°, 27.6°±0.2°;
Crystal form III: 6.8°±0.2°, 13.5°±0.2°, 20.3°±0.2°, 21.3°±0.2°;
Crystal form IV: 6.2°±0.2°, 6.6°±0.2°, 10.2°±0.2°, 14.1°±0.2°.

Crystal forms of the compound of formula (1) have X-ray powder diffraction patterns comprising the following characteristic peaks expressed by 20 degree, when measured using Cu-Kalpha radiation:
Crystal form I: 6.3°±0.2°, 9.6°±0.2°, 10.9°±0.2°, 12.6°±0.2°, 19.0°±0.2°, 21.0°±0.2°;
Crystal form II: 6.7°±0.2°, 11.0°±0.2°, 13.3°±0.2°, 15.5°±0.2°, 17.1°±0.2°, 27.6°±0.2°;
Crystal form III: 6.8°±0.2°, 9.7°±0.2°, 11.1°±0.2°, 13.5°±0.2°, 20.3°±0.2°, 21.3°±0.2°;
Crystal form IV: 6.2°±0.2°, 6.6°±0.2°, 10.2°±0.2°, 12.5°±0.2°, 14.1°±0.2°, 19.3°±0.2°.

Crystal forms of the compound of formula (1) have X-ray powder diffraction patterns comprising the following characteristic peaks expressed by 20 degree, when measured using Cu-Kalpha radiation:
Crystal form I: 6.3°±0.2°, 9.6°±0.2°, 10.9°±0.2°, 12.6°±0.2°, 14.9°±0.2°, 19.0°±0.2°, 21.0°±0.2°, 24.4°±0.2°, 29.1°±0.2°, 30.1°±0.2°; or
Crystal form I: 6.3°±0.2°, 9.6°±0.2°, 10.9°±0.2°, 12.6°±0.2°, 14.9°±0.2°, 19.0°±0.2°, 21.0°±0.2°, 24.4°±0.2°, 26.1°±0.2°, 29.1°±0.2°; or
Crystal form I: 6.3°±0.2°, 9.6°±0.2°, 10.9°±0.2°, 12.6°±0.2°, 14.9°±0.2°, 19.0°±0.2°, 21.0°±0.2°, 24.4°±0.2°, 26.1°±0.2°, 29.1°±0.2°, 30.1°±0.2°;
Crystal form II: 6.7°±0.2°, 11.0°±0.2°, 13.3°±0.2°, 15.5°±0.2°, 17.1°±0.2°, 20.0°±0.2°, 24.1°±0.2°, 27.6°±0.2°, 29.4°±0.2°, 31.6°±0.2°;
Crystal form III: 6.8°±0.2°, 9.7°±0.2°, 11.1°±0.2°, 13.5°±0.2°, 20.3°±0.2°, 21.3°±0.2°, 27.3°±0.2°, 27.6°±0.2°, 29.2°±0.2°, 31.4°±0.2°;
Crystal form IV: 6.2°±0.2°, 6.6°±0.2°, 9.3°±0.2°, 10.2°±0.2°, 12.5°±0.2°, 14.1°±0.2°, 15.5°±0.2°, 17.0°±0.2°, 19.3°+0.2°, 27.6°±0.2°.

Crystal forms of the compound of formula (1) have X-ray powder diffraction patterns comprising the following characteristic peaks expressed by 2θ degree, when measured using Cu-Kalpha radiation:
Crystal form I: 6.3°±0.1°, 9.6°±0.1°, 10.9°±0.1°, 12.6°±0.1°, 14.9°±0.1°, 19.0°±0.1°, 21.0°±0.1°, 24.4°±0.1°, 29.1°±0.1°, 30.1°±0.1°; or
Crystal form I: 6.3°±0.1°, 9.6°±0.1°, 10.9°±0.1°, 12.6°±0.1°, 14.9°±0.1°, 19.0°±0.1°, 21.0°±0.1°, 24.4°±0.1°, 26.1°±0.1°, 29.1°±0.1°; or
Crystal form I: 6.3°±0.1°, 9.6°±0.1°, 10.9°±0.1°, 12.6°±0.1°, 14.9°±0.1°, 19.0°±0.1°, 21.0°±0.1°, 24.4°±0.1°, 26.1°±0.1°, 29.1°±0.1°, 30.1°±0.1°;
Crystal form II: 6.7°±0.1°, 11.0°±0.1°, 13.3°±0.1°, 15.5°±0.1°, 17.1°±0.1°, 20.0°±0.1°, 24.1°±0.1°, 27.6°±0.1°, 29.4°±0.1°, 31.6°±0.1°;
Crystal form III: 6.8°±0.1°, 9.7°±0.1°, 11.1°±0.1°, 13.5°±0.1°, 20.3°±0.1°, 21.3°±0.1°, 27.3°±0.1°, 27.6°±0.1°, 29.2°±0.1°, 31.4°±0.1°;
Crystal form IV: 6.2°±0.1°, 6.6°±0.1°, 9.3°±0.1°, 10.2°±0.1°, 12.5°±0.1°, 14.1°±0.1°, 15.5°±0.1°, 17.0°±0.1°, 19.3°±0.1°, 27.6°±0.1°.

Crystal forms of the compound of formula (1) are characterized in that:
The differential scanning calorimetry thermogram of crystal form I shows an endothermic peak in the range of 270-295°C, preferably, the onset temperature of the endothermic peak is 284.5°C, and the peak temperature is 285.1°C;
The differential scanning calorimetry thermogram of crystal form II shows an endothermic peak in the range of 275-293°C, preferably, the onset temperature of the endothermic peak is 283.6°C, and the peak temperature is 285.2°C;
The differential scanning calorimetry thermogram of crystal form III shows an endothermic peak in the range of 270-295°C, preferably, the onset temperature of the endothermic peak is 284.0°C, and the peak temperature is 285.4°C.

The crystal forms of the compound of formula (1) are characterized in that,
Crystal form I: the X-ray powder diffraction pattern as shown in Figure 1;
Crystal form II: the X-ray powder diffraction pattern as shown in Figure 2;
Crystal form III: the X-ray powder diffraction pattern as shown in Figure 3; and
Crystal form IV: the X-ray powder diffraction pattern as shown in Figure 4.

The present invention also provides preparation methods for crystal forms I, II, III and IV of the compound of formula (1), comprising the following steps:
placing the compound of formula (1) in a solvent such as lower alcohols, tetrahydrofuran, or a mixed solution of acetonitrile and water, and obtaining crystal form I via a method including suspending-slurry washing, volatilizing, and/or cooling;
placing the compound of formula (1) in a solvent such as anhydrous lower alcohols or tetrahydrofuran, heating the mixture until dissolved, cooling to obtain crystal form II;
placing the compound of formula (1) in a lower alcohol, heating the mixture until dissolved, adding water thereto, cooling to 20°C or less, and filtering to obtain crystal form III; or
heating crystal form I of the compound of formula (1) to 100°C or higher to obtain metastable crystal form V; placing crystal form V at ambient temperature and humidity to transform into crystal form IV.

The preparation methods for crystal forms I, II, III, and IV of the compound of formula (1) particularly comprise the following steps:
placing the compound of formula (1) in lower alcohols or tetrahydrofuran or a mixed solution of acetonitrile and water, preferably tetrahydrofuran, and obtaining crystal form I via a method including suspending-slurry washing, volatilizing, and/or cooling, preferably via heating the compound until dissolved (e.g., to 50-60°C, e.g. 55°C), cooling (e.g., to 20-40°C, e.g. 30°C), and vacuum drying at 20-40°C, e.g. 30°C, for 1-24 hours (e.g., 4-8 hours);
placing the compound of formula (1) in a solvent such as anhydrous lower alcohols or tetrahydrofuran, preferably tetrahydrofuran, and heating the mixture until dissolved and then cooling, preferably heating the compound until dissolved (e.g., to 50-60°C, e.g. 55°C) and then cooling to 15-25°C (e.g. room temperature of 25°C), filtering, and vacuum drying the resulting solid at 45-55°C, e.g. 50°C, for 1-24 hours (e.g., 4-8 hours) to obtain crystal form II;
placing the compound of formula (1), e.g. the compound of crystal form I, in a lower alcohol (e.g., C1-3alcohol such as methanol, ethanol, and propanol), heating the mixture until dissolved (e.g., to 50-60°C, e.g. 55°C), optionally filtering, and placing the obtained solution or filtrate at 15-25°C (e.g., room temperature 25°C) to allow the solvent to slowly volatilize to produce crystal form III; or placing the compound of formula (1), e.g. the compound of crystal form I, in a mixture of a lower alcohol (e.g., C₁₋₆alcohol, such as methanol, ethanol, and propanol) and water (alcohol:water mass ratio=1:5-5:1, e.g., 1:3), cooling the mixture to 20°C or lower (e.g., 5-20°C, such as 5°C), adding a crystal seed of crystal form III, stirring the mixture for 1-24 hours (e.g., 4-8 hours), then filtering, and vacuum drying the obtained solid at 25-40°C, e.g. 30°C, for 1-24 hours (e.g., 4-8 hours) to obtain crystal form III;
heating crystal form I of the compound of formula (1) to 100°C or higher (for example 100-150°C, e.g. 100°C) to obtain a metastable crystal form V; placing crystal form V at ambient temperature (for example 15-35°C, e.g. 25-30°C) and under a certain humidity (for example RH=30-90%, e.g. RH=70-85%) for 1-24 hours (e.g., 4-8 hours) to transform into crystal form IV.

The present invention also provides a pharmaceutical composition comprising the crystal form of the compound of formula (1) and a pharmaceutically acceptable carrier, wherein said crystal form includes crystal forms I, II, III and IV, and a combination thereof. Based on the weight of the pharmaceutical composition, the crystal form of the compound of formula (1) comprises 1-99wt%, for example, 20-80wt%, 30-70wt%, or 45-55wt%, and the pharmaceutically acceptable carrier comprises 99-1wt%, for example, 80-20wt%, 70-30wt%, or 55-45wt%.

In the pharmaceutical composition of the present invention, said crystal form exists in pure form, which includes but is not limited to, the content of crystal form I, II or III, or a combination thereof being not less than 94.5wt% or 95wt% or 96wt% or 98wt% or 99wt%, and for example, the presence of less than about 5.5wt%, less than about 5wt%, less than about 4%, less than about 2%, or less than about 1% of impurities. Such impurities include but are not limited to degradation products, oxidation products, epimers, solvents, and/or other undesirable impurities.

The present invention also provides a pharmaceutical formulation comprising the crystal form of the compound of formula (1) and one or more pharmaceutically acceptable carriers and/or diluents, in any pharmaceutically acceptable dosage form. In the pharmaceutical formulation of the present invention, the crystal form of the compound of formula (1) comprises 1-99wt%, for example, 20-80wt%, 30-70wt%, or 45-55wt%, and the pharmaceutically acceptable carrier and/or diluent comprises 99-1wt%, for example, 80-20wt%, 70-30wt%, or 55-45wt%. The present invention also provides that the crystal form of the compound of formula (1) can be made into a pharmaceutical formulation with one or more pharmaceutically acceptable carriers. Said pharmaceutical formulation refers to conventional preparations used clinically, which can be administered to patients in need of such treatment by oral or parenteral administration, etc. Oral administration is preferred, and the pharmaceutical formulation is preferably an oral preparation. Said pharmaceutical formulation includes conventional solid formulations, conventional liquid formulations, and the like. For oral administration, said crystal form can be made into conventional solid formulations such as tablets, capsules, pills, granules, etc.; it can also be made into oral liquid formulations such as oral solutions, oral suspensions, syrups, etc. For parenteral administration, it can be made into injectable preparations, including injections, sterile powders for injection, concentrated solutions for injection, suspensions for injection, etc. For rectal administration, it can be made into suppositories, etc. For pulmonary administration, it can be made into inhalation preparations, spray preparations, etc. For topical or transdermal administration, it can be made into ointments, pastes, creams, lotions, gels, powders, solutions, or transdermal patches, etc. These formulations can be prepared by conventional methods, for example by adding pharmaceutically acceptable carriers such as excipients, binders, humectants, disintegrants, thickeners, etc; wherein said crystal form includes crystal forms I, II, III and IV, and combinations thereof.

The present invention also provides use of the crystal form of the compound of formula (1) in manufacture of medicament for treating and/or preventing uratic and/or gouty diseases, wherein said crystal form includes crystal forms I, II, III and IV, and combinations thereof.

Compared with the prior art, the crystal forms of the compound of formula (1) of the present invention have the following advantages:
(1) Crystal forms I, II, III and IV of the compound of formula (1) of the present invention, or combinations thereof have good stability. Among them, the stability of crystal form I is better than that of crystal forms II, III, and IV, and the thermal stability of crystal form I is better than that of crystal forms II, III, and IV;
(2) Crystal forms I, II, III and IV of the compound of formula (1) of the present invention, or combinations thereof exhibit low residual solvent and low toxicity and side effects.
(3) Crystal forms I, II, III and IV of the compound of formula (1) of the present invention, or combinations thereof have good physicochemical properties, stable quality, and uniform particle size distribution, and are easy for large-scale industrial production.
(4) Crystal forms I, II, III and IV of the compound of formula (1) of the present invention, or combinations thereof are particularly suitable for the preparation of pharmaceuticals aimed at lowering uric acid, reducing inflammation, and/or treating and/or preventing uratic and/or gouty diseases, and can enhance patient compliance.

Experiments showed that for pharmaceutical applications, crystal forms I and II were advantageous in terms of thermodynamic stability, and crystal form III was suboptimal. It was also found through the study on crystallization kinetics that for pharmaceutical applications, crystal form I was more advantageous. According to the study on kinetic stability in the solid state, when stored at 10°C-30°C in double-sealed aluminum packaging and plastic self-sealing bags for more than 6 years, crystal form I remained unchanged. This result indicated that crystal form I had good kinetic stability in the solid state. The dehydration risk of the monohydrate crystal form was also evaluated by using VT-XRPD (variable temperature X-ray powder diffraction) in the present invention. The results showed that the dehydration risk of crystal form I was very low. The stability of crystal form I in the formulation was also evaluated in the present invention. After stored at 40°C/75% RH for 6 months or 25°C/60% RH for 9 months, the content of crystal form III in the formulation was below LOD (limit of detection), indicating that crystal form I also showed good physical stability in the formulation without transformation into crystal form III. The stability of crystal forms I and IV in the solid-state was also studied in the present invention. The results indicated that after storage under accelerated conditions (25°C/92.5% RH, open container) or (40°C/75% RH, open container) for one month, crystal form I did not transform, while crystal form IV transformed into crystal form I.

In conclusion, crystal form I has relatively high crystallinity, relatively high dehydration temperature, and good manufacturability. Its solid maintains good kinetic stability when stored at ambient temperature for several years. It shows good stability in the formulation. Therefore, the risk of polymorphic transformation for crystal form I is very small.

Crystal forms I, II, III and IV of the compound of formula (1) of the present invention, or combinations thereof are characterized in that physical properties such as stability, solubility, hygroscopicity, and dissolution rate are suitable for clinical and therapeutic dosage forms; they are also characterized in that physical properties such as crystal morphology, compressibility, particle size, and hardness are suitable for preparing solid dosage forms. The above properties can be determined using techniques known in the art, such as X-ray diffraction, microscopy, IR spectroscopy, thermal analysis, and hygroscopicity analysis. The crystal form of a compound can affect its solubility, dissolution rate, bioavailability, chemical and physical stability, flowability, breakability, and compressibility, as well as the safety and efficacy of drug products based on the compound. Therefore, it becomes very important to prepare and market pure drugs existing in their thermodynamically most stable crystal form and being substantially free of other crystal forms.

The crystal form of a compound with optimal physical and chemical properties will facilitate the development of the active compound as a drug. The most useful physical and chemical properties include: ease and reproducibility of manufacture, crystallinity, non-hygroscopicity, water solubility, stability to visible and ultraviolet light, low degradation rate under accelerated stability conditions of temperature and humidity, low isomerization rate between isomeric forms, and long-term safety in humans, etc.

Crystal forms I, II, III and IV, and combinations thereof of the compound of formula (1) of the present invention exhibit storage stability, compressibility, density, particle size stability, dissolution properties, and other characteristics that are advantageous for preparing and formulating the compound of formula (1), and using it in the formulation, as well as for its bioavailability.

Crystal forms I, II, III, IV, and V of the compound of formula (1) of the present invention (including mixed crystals thereof) can undergo mutual transformation under specific conditions. The present invention further discloses the transformation relationships among crystal form I, crystal form II, crystal form III, crystal form IV, and crystal form V.

### Description of the drawings

Figure 1: XRPD pattern of crystal form I of the compound of formula (1);
Figure 2: XRPD pattern of crystal form II of the compound of formula (1);
Figure 3: XRPD pattern of crystal form III of the compound of formula (1);
Figure 4: XRPD pattern of crystal form IV of the compound of formula (1);
Figure 5: XRPD pattern of crystal form V of the compound of formula (1);
Figure 6: DSC thermogram of crystal form I of the compound of formula (1);
Figure 7: TGA curve of crystal form I of the compound of formula (1);
Figure 8: DSC thermogram of crystal form II of the compound of formula (1);
Figure 9: TGA curve of crystal form II of the compound of formula (1);
Figure 10: DSC thermogram of crystal form III of the compound of formula (1);
Figure 11: TGA curve of crystal form III of the compound of formula (1);
Figure 12: DSC thermogram of crystal form IV of the compound of formula (1);
Figure 13: TGA curve of crystal form IV of the compound of formula (1);
Figure 14: Transformation relationship among crystal form I, crystal form II, crystal form III, crystal form IV, and crystal form V of the compound of formula (1), wherein:
   1. Rotary evaporation of a solution of crystal form I in tetrahydrofuran may produce crystal form II; or lyophilization of crystal form I in 1,4-dioxane may produce crystal form II;
   2. Suspending and separating in cumene at 5°C and 25°C;
   3. Heating to 95°C, then cooling to ambient conditions in a lower alcohol or ketone aqueous solution (a.w. ≤ 0.3) at 10-40°C;
   4. Suspending and separating in nitromethane at 5°C and 25°C;
   5. Slow volatilization in methanol; rapid cooling in tetrahydrofuran and water (1:1, v/v);
   6. Placing in a methanol/water system (a.w.≥0.4) at 30-40°C;
   7. Completely dehydrating crystal form I at 100°C to transform to crystal form V; drying crystal form I at 60°C under vacuum for 24 hours to transform to crystal form V;
   8. Suspending and separating in acetone/water system (a.w.≥0.2) at room temperature, in acetone/water system (a.w.≥0.4) or methanol/water system (a.w.≥0.4) at 50°C, or in acetonitrile/water system (a.w.≥0.6) or water at 70°C;
   9. Heating to 150°C then cooling to ambient conditions;
   10. Transformation after placing at 25°C/92.5% RH or 40°C/75% RH for one month, or placing at 25°C/60% RH for 30 days;
   11. Transformation of crystal form V to crystal form IV after placing under ambient conditions (25-30°C, 70-85% RH) for about 10 hours;
   12. placing at 60°C for 5 days or for one month;
   13. Suspending and separating in ethyl acetate/acetone at room temperature; suspending and separating in an acetone/water system (a.w.=0-0.2) at 50°C; or suspending and separating in acetonitrile/water system (a.w.=0-0.4) at 70°C.

The term "slurry washing" herein refers to stirring and washing a large amount of solid with a small amount of solvent.

The term "lower alcohol" herein refers to alcohols containing 1-6 carbon atoms, including methanol, ethanol, propanol, isopropanol, butanol, isobutanol, n-butanol, pentanol, hexanol, and the like.

### Detailed description of the embodiments

The following specific embodiments in the form of examples provide further detailed description of the above contents of the present invention. However, this should not be construed as limiting the scope of the above subject matters of the present invention to the following examples. Any technical solutions implemented based on the above contents of the present invention belong to the scope of the present invention.

### Example 1: Preparation of 8-chloro-3-pentyl-3,7-dihydro-1H-purine-2,6-dione

Referring to the method described in WO2016/119570A1, the compound 8-chloro-3-pentyl-3,7-dihydro-1H-purine-2,6-dione monohydrate (60g) was synthesized in a yield of 67.7%.

Molecular formula: C₁₀H₁₃ClN₄O₃; molecular weight: 274.1; mass spectrum (M+H): 257.1

¹H-NMR(DMSO-*d₆*, 400MHz): 0.84-0.86(t, 3H), 1.28(m, 4H), 1.63(m, 2H), 3.85(t, 2H), 11.22(s, 1H), 14.38(br.s, 1H).

### Example 2: Preparation of crystal form I of the compound 8-chloro-3-pentyl-3,7-dihydro-1H-purine-2,6-dione

10g of the compound of formula (1) prepared in Example 1 was placed in 150g of tetrahydrofuran. The mixture was heated to 55°C with stirring until dissolved. 150g of water was added, and the mixture was cooled to 30°C and filtered. The resulting solid was placed in a vacuum drying oven and dried under vacuum at 30°C overnight to produce crystal form I.

According to the Karl Fischer (KF) detection result, it contained about 1.1 equivalents (7.6wt%) of water. DSC showed that crystal form I began to dehydrate at about 30°C. TGA showed a weight loss of about 6.6% at 100°C. The melting point was 284.5°C.

The X-ray powder diffraction (XRPD) pattern of crystal form I is shown in Figure 1 with the main parameters as follows:

| **2θ angles (°)** | **Intensity (%)** | **d-value (Å)** |
|---|---|---|
| 6.326° | 100.0% | 13.95950Å |
| 9.633° | 13.0% | 9.17426Å |
| 10.854° | 8.6% | 8.14470Å |
| 12.639° | 10.8% | 6.99790Å |
| 14.934° | 6.8% | 5.92745Å |
| 16.822° | 1.9% | 5.26632Å |
| 18.993° | 23.0% | 4.66893Å |
| 20.218° | 0.6% | 4.38861Å |
| 21.045° | 12.8% | 4.21803Å |
| 24.405° | 4.6% | 3.64430Å |
| 25.459° | 2.4% | 3.49586Å |
| 25.942° | 2.7% | 3.43183Å |
| 26.069° | 4.3% | 3.41542Å |
| 28.482° | 1.5% | 3.13128Å |
| 29.097° | 7.2% | 3.06645Å |
| 30.072° | 3.7% | 2.96925Å |
| 30.964° | 1.2% | 2.88570Å |
| 36.469° | 2.1% | 2.46174Å |

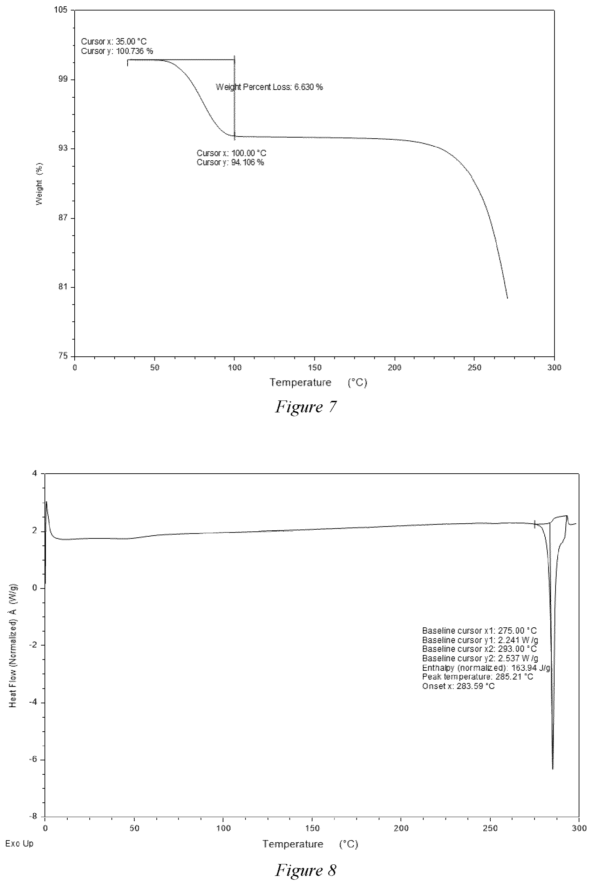

The DSC thermogram and the TGA curve of crystal form I of the compound of formula (1) are shown in Figures 6 and 7 respectively.

### Example 3: Preparation of crystal form II of the compound 8-chloro-3-pentyl-3,7-dihydro-1H-purine-2,6-dione

5g of the compound of formula (1) prepared in Example 1 was placed in 50g of tetrahydrofuran. The mixture was heated to 55°C with stirring until dissolved, cooled to room temperature and filtered. The resulting solid was placed in a vacuum drying oven and dried under vacuum at 50°C overnight to produce crystal form II. The melting point was 283.6°C.

The X-ray powder diffraction (XRPD) pattern of crystal form II is shown in Figure 2 with the main parameters as follows:

| **2θ angles (°)** | **Intensity (%)** | **d-value (Å)** |
|---|---|---|
| 6.652° | 100.0% | 13.27774Å |
| 9.387° | 0.5% | 9.41384Å |
| 10.986° | 3.5% | 8.04693Å |
| 12.028° | 0.8% | 7.35223Å |
| 13.283° | 3.0% | 6.66021Å |
| 15.546° | 14.8% | 5.69560Å |
| 17.057° | 2.9% | 5.19418Å |
| 19.998° | 1.5% | 4.43639Å |
| 21.285° | 1.3% | 4.17097Å |
| 22.056° | 1.0% | 4.02686Å |
| 22.936° | 0.8% | 3.87441Å |
| 24.066° | 1.4% | 3.69500Å |
| 26.631° | 0.7% | 3.34462Å |
| 27.562° | 3.5% | 3.23368Å |
| 29.385° | 2.0% | 3.03713Å |
| 31.629° | 1.5% | 2.82655Å |
| 32.575° | 1.2% | 2.74657Å |
| 34.598° | 1.0% | 2.59048Å |
| 37.208° | 0.5% | 2.41452Å |
| 38.005° | 0.6% | 2.36574Å |

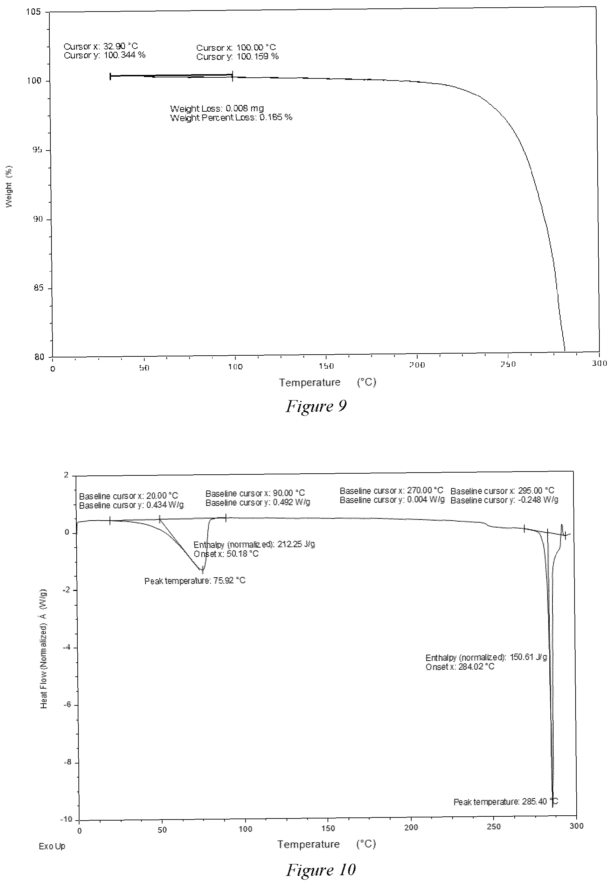

The DSC thermogram and the TGA curve of crystal form II of the compound of formula (1) are shown in Figures 8 and 9 respectively.

### Example 4: Preparation of crystal form III of the compound 8-chloro-3-pentyl-3,7-dihydro-1H-purine-2,6-dione

Method 1: 50 mg of crystal form I prepared in Example 2 was completely dissolved in 10 mL of methanol, filtered, and subjected to slow evaporation at ambient temperature. The solid obtained from evaporation was collected as crystal form III.

Method 2: 1 g of crystal form I prepared in Example 2 was placed in a mixed solvent of 10 g of methanol and water (methanol/water=1:3, w/w). The mixture was cooled to 5°C and stirred for 1-2 hours. Subsequently, 10 mg of crystal form III was added, and the stirring was continued overnight. The mixtue was filtered. The resulting solid was placed in a vacuum drying oven and dried under vacuum at 30°C overnight to produce crystal form III. The melting point was 284.0°C.

The X-ray powder diffraction (XRPD) pattern of crystal form III is shown in Figure 3 with the main parameters as follows:

| **2θ angles (°)** | **Intensity (%)** | **d-value (Å)** |
|---|---|---|
| 6.752° | 100.0% | 13.07994Å |
| 9.678° | 9.9% | 9.13149Å |
| 11.068° | 9.8% | 7.98741Å |
| 13.492° | 12.2% | 6.55774Å |
| 15.569° | 4.9% | 5.68691Å |
| 17.594° | 2.5% | 5.03685Å |
| 19.389° | 1.2% | 4.57432Å |
| 20.282° | 29.2% | 4.37502Å |
| 21.343° | 13.0% | 4.15982Å |
| 24.350° | 2.9% | 3.65251Å |
| 25.070° | 3.3% | 3.54924Å |
| 26.345° | 5.0% | 3.38029Å |
| 27.309° | 6.4% | 3.26306Å |
| 27.645° | 6.6% | 3.22416Å |
| 29.071° | 2.4% | 3.06918Å |
| 29.217° | 5.9% | 3.05415Å |
| 29.706° | 1.0% | 3.00502Å |
| 31.403° | 5.3% | 2.84634Å |
| 32.945° | 1.6% | 2.71658Å |
| 33.554° | 1.7% | 2.66865Å |
| 34.143° | 1.9% | 2.62395Å |
| 36.764° | 1.5% | 2.44269Å |
| 37.552° | 2.0% | 2.39324Å |

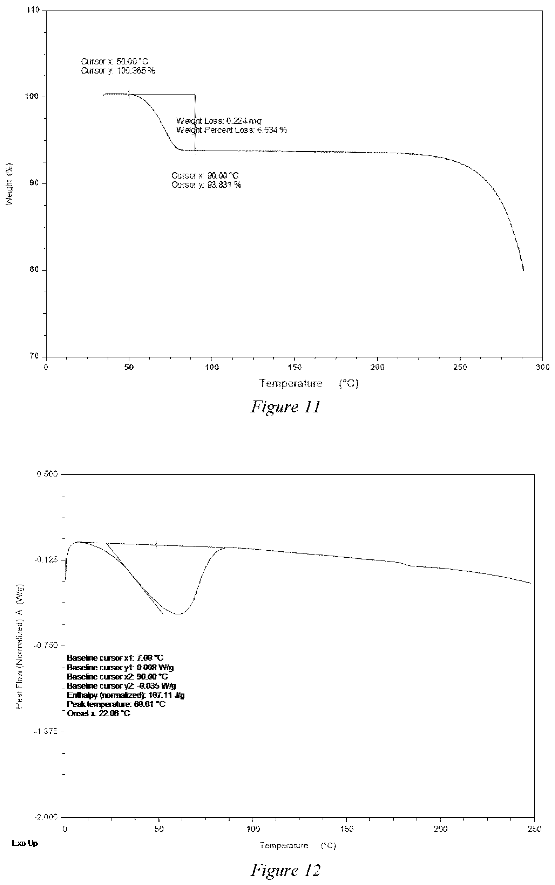

The DSC thermogram and the TGA curve of crystal form III of the compound of formula (1) are shown in Figures 10 and 11 respectively.

### Example 5: Preparation of crystal form IV of the compound 8-chloro-3-pentyl-3,7-dihydro-1H-purine-2,6-dione

1 g of crystal form I prepared in Example 2 was completely dehydrated at 100°C. The resulting material was then placed under ambient conditions (25-30°C and 70-85% RH) for about 10 hours, thereby being transformed to crystal form IV.

The X-ray powder diffraction (XRPD) pattern of crystal form IV is shown in Figure 4 with the main parameters as follows:

| **2θ angles (°)** | **Intensity (%)** | **d-value (Å)** |
|---|---|---|
| 6.241° | 100.0% | 14.14955Å |
| 6.609° | 25.2% | 13.36430Å |
| 9.309° | 3.7% | 9.49232Å |
| 10.167° | 20.2% | 8.69381Å |
| 10.976° | 2.8% | 8.05436Å |
| 11.993° | 0.7% | 7.37345Å |
| 12.152° | 2.0% | 7.27741Å |
| 12.461° | 5.7% | 7.09778Å |
| 13.248° | 0.6% | 6.67770Å |
| 14.057° | 7.4% | 6.29501Å |
| 15.520° | 4.8% | 5.70496Å |
| 16.941° | 4.1% | 5.22955Å |
| 18.513° | 1.8% | 4.78880Å |
| 19.252° | 5.6% | 4.60662Å |
| 20.799° | 1.4% | 4.26739Å |
| 22.022° | 0.7% | 4.03303Å |
| 23.826° | 1.8% | 3.73161Å |
| 24.444° | 2.7% | 3.63869Å |
| 27.240° | 0.6% | 3.27117Å |
| 27.579° | 3.0% | 3.23176Å |
| 28.320° | 1.4% | 3.14882Å |
| 30.751° | 0.8% | 2.90518Å |
| 31.428° | 1.4% | 2.84420Å |
| 32.508° | 0.7% | 2.75210Å |
| 37.173° | 0.9% | 2.41671Å |
| 38.182° | 0.8% | 2.35513Å |

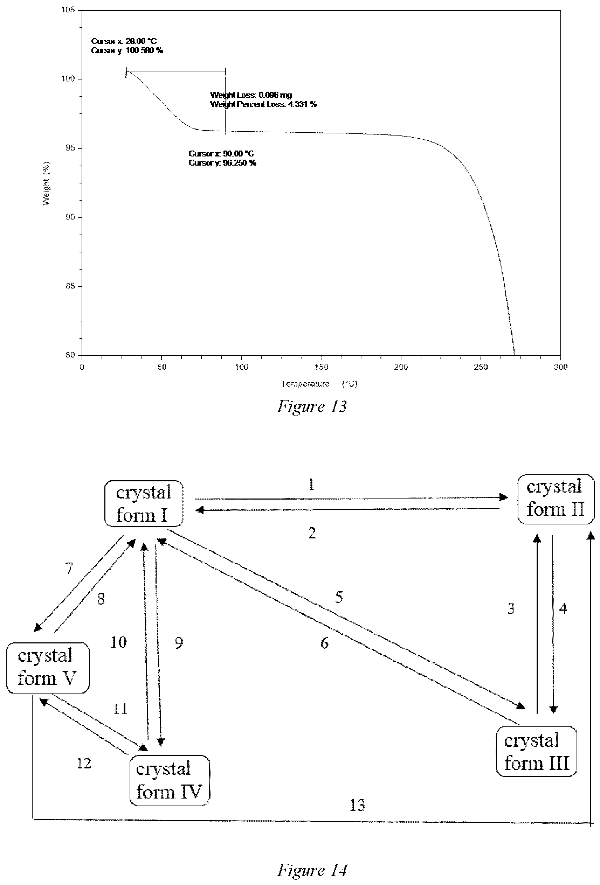

The DSC thermogram and the TGA curve of crystal form IV of the compound of formula (1) are shown in Figures 12 and 13 respectively.

The beneficial effects of the compound of the present invention are further elaborated below through stability experiments on various crystal forms; however, this should not be construed as the compound of the present invention having only the following beneficial effects.

### Test Example 1: Stability of various crystal forms of the compound of the present invention

Test samples: crystal form I and crystal form II of the compound of formula (1) were prepared according to the above examples.

### Test conditions for investigating the influencing factors:

High humidity test: Crystal form I and crystal form II of the compound of formula (1) were laid on a dry and clean watch glass, and kept at 40°C±2°C and 75% RH±5% for 10 days. Samples were taken respectively on Day 5 and Day 10. The purity and the related substance content of the compound of formula (1) were determined and compared with those of the sample taken on Day 0.

Photostablity test: Crystal form I and crystal form II of the compound of formula (1) were laid on a dry and clean watch glass, and kept at an illuminance of 5000Lx±500Lx in a light box for 10 days. Samples were taken respectively on Day 5 and Day 10. The purity and the related substance content of the compound of formula (1) were determined and compared with those of the sample taken on Day 0.

Test results were shown in Table 1 below.

**Table 1: The investigation results of the influencing factor tests for crystal form I and crystal form II of the compound of formula (1)**

| | | Crystal form I | | Crystal form II | |
|---|---|---|---|---|---|
| Conditions | Day | Purity (%) | Related substance (%) | Purity (%) | Related substance (%) |
| / | 0 | 99.6 | 0.45 | 99.7 | 0.32 |
| 40°C±2°C/ | 5 | 99.5 | 0.46 | 99.7 | 0.31 |
| 75%±5%RH | 10 | 99.6 | 0.42 | 99.7 | 0.32 |
| illuminance | 5 | 99.6 | 0.44 | 99.7 | 0.31 |
| 5000Lx±500Lx | 10 | 99.5 | 0.46 | 99.7 | 0.32 |

The test results demonstrated that the purities/related substance contents of crystal forms I and II of the compound of formula (1) changed very little under high humidity and light exposure, indicating high stability, which facilitates drug preparation, storage, and transportation, thereby ensuring the drug's efficacy and safety.

### Test Example 2: in vivo PK characteristics of crystal forms of the compound of the present pnvention in SD rats

Test samples: the compound of represented by formula (1), and crystal forms I and II of the compound of formula (1) were prepared according to Examples 1-3;
The tris (i.e. tris(hydroxymethyl)aminomethane) salt crystal form of the compound 8-chloro-3-pentyl-3,7-dihydro-1H-purine-2,6-dione was prepared with reference to WO2010/068581A1.

Oral administration (PO) of four samples:

| **Number of Animals** | **Sex** | **Route** | **Dose (mg/kg)** | **Volume (mL/kg)** | **Blood Collection Time Points** | **Biological Sample Type** |
|---|---|---|---|---|---|---|
| 3 per sample | Male | PO | 2 | 2 | 10min, 30min, 1h, 2h, 4h, 6h, 8h, 24h | Plasma |

### Data processing method:

The concentrations of the test substances were output using Analyst 1.6.2 from AB Sciex. Parameters such as mean, standard deviation, and variation coefficient were calculated using Microsoft Excel (those directly output by Analyst 1.6.2 were not recalculated). PK parameters were calculated using Pharsight Phoenix 6.2 software (NCA model).

Test results were shown in Table 2 below.

**Table 2: Summary of in vivo PK parameters after oral administration in SD rats (mean±SD) (n=3)**

| **Sample** | **Solid product of Example 1** | **Crystal form I** | **Crystal form II** | **tris salt crystal form** |
|---|---|---|---|---|
| t_{1/2}(h) | 2.32±0.09 | 5.28±1.10 | 4.26±0.61 | 2.38±0.17 |

The test results demonstrated that crystal forms I and II of the present invention exhibited longer half-lifes (t_{1/2}).

### Test Example 3: Hygroscopicity test (according to Chinese Pharmacopoeia, Guidelines for Drug Hygroscopicity Testing, at 25±1°C and 80%±2% Relative Humidity)

An appropriate amount of the sample from the examples was taken for the hygroscopicity test. The results were as follows:

| Samples | | Hygroscopicity test result | |
|---|---|---|---|
| | | Weight gain due to hygroscopicity | Hygroscopicity |
| Example 2 | Crystal form I | <0.2% | Non-hygroscopic or almost non-hygroscopic |
| Example 3 | Crystal form II | <0.2% | Non-hygroscopic or almost non-hygroscopic |

The test results demonstrated that crystal forms I and II of the present invention exhibited extremely low hygroscopicity.

Additionally, a dynamic vapor sorption (DVS) instrument was used to further study the hygroscopicity of crystal forms I, II, III and IV, and the method of Test Example 1 was used to study the stability of crystal forms III and IV. The comparative study demonstrated that crystal forms I and II were more advantageous for pharmaceutical applications.

## Claims

1. A crystal form of the compound 8-chloro-3-pentyl-3,7-dihydro-1H-purine-2,6-dione represented by formula (1), 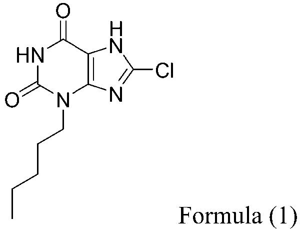 which is **characterized by** having an X-ray powder diffraction pattern comprising the following characteristic peaks expressed by 2θ degree, when measured using Cu-Kalpha radiation:
Crystal form I: 6.3°±0.2°, 9.6°±0.2°, 19.0°±0.2°, 21.0°±0.2°;
Crystal form II: 6.7°±0.2°, 11.0°±0.2°, 15.5°±0.2°, 27.6°±0.2°;
Crystal form III: 6.8°±0.2°, 13.5°±0.2°, 20.3°±0.2°, 21.3°±0.2°;
Crystal form IV: 6.2°±0.2°, 6.6°±0.2°, 10.2°±0.2°, 14.1°±0.2°.

2. The crystal form of the compound of formula (1) according to claim 1, which is **characterized by** having an X-ray powder diffraction pattern comprising the following characteristic peaks expressed by 20 degree, when measured using Cu-Kalpha radiation:
Crystal form I: 6.3°±0.2°, 9.6°±0.2°, 10.9°±0.2°, 12.6°±0.2°, 19.0°±0.2°, 21.0°±0.2°;
Crystal form II: 6.7°±0.2°, 11.0°±0.2°, 13.3°±0.2°, 15.5°±0.2°, 17.1°±0.2°, 27.6°±0.2°;
Crystal form III: 6.8°±0.2°, 9.7°±0.2°, 11.1°±0.2°, 13.5°±0.2°, 20.3°±0.2°, 21.3°±0.2°;
Crystal form IV: 6.2°±0.2°, 6.6°±0.2°, 10.2°±0.2°, 12.5°±0.2°, 14.1°±0.2°, 19.3°±0.2°.

3. The crystal form of the compound of formula (1) according to claim 1 or 2, which is **characterized by** having an X-ray powder diffraction pattern comprising the following characteristic peaks expressed by 2θ degree, when measured using Cu-Kalpha radiation:
Crystal form I: 6.3°±0.2°, 9.6°±0.2°, 10.9°±0.2°, 12.6°±0.2°, 14.9°±0.2°, 19.0°±0.2°, 21.0°±0.2°, 24.4°±0.2°, 29.1°±0.2°, 30.1°±0.2°;
Crystal form II: 6.7°±0.2°, 11.0°±0.2°, 13.3°±0.2°, 15.5°±0.2°, 17.1°±0.2°, 20.0°±0.2°, 24.1°±0.2°, 27.6°±0.2°, 29.4°±0.2°, 31.6°±0.2°;
Crystal form III: 6.8°±0.2°, 9.7°±0.2°, 11.1°±0.2°, 13.5°±0.2°, 20.3°±0.2°, 21.3°±0.2°, 27.3°±0.2°, 27.6°±0.2°, 29.2°±0.2°, 31.4°±0.2°;
Crystal form IV: 6.2°±0.2°, 6.6°±0.2°, 9.3°±0.2°, 10.2°±0.2°, 12.5°±0.2°, 14.1°±0.2°, 15.5°±0.2°, 17.0°±0.2°, 19.3°+0.2°, 27.6°±0.2°.

4. The crystal form of the compound of formula (1) according to any one of claims 1-3, which is **characterized in that**,
crystal form I has an X-ray powder diffraction pattern as shown in Figure 1;
crystal form II has an X-ray powder diffraction pattern as shown in Figure 2;
crystal form III has an X-ray powder diffraction pattern as shown in Figure 3; and
crystal form IV has an X-ray powder diffraction pattern as shown in Figure 4.

5. The crystal form of the compound of formula (1) according to any one of claims 1-4, which is **characterized in that**,
crystal form I has an endothermic peak in the range of 270-295°C in its differential scanning calorimetry thermogram;
crystal form II has an endothermic peak in the range of 275-293°C in its differential scanning calorimetry thermogram; and
crystal form III has an endothermic peak in the range of 270-295°C in its differential scanning calorimetry thermogram.

6. A method for preparing the crystal form of the compound of formula (1) according to any one of claims 1-5, **characterized in that**,
placing the compound of formula (1) in lower alcohols, tetrahydrofuran, or a mixed solution of acetonitrile and water, and obtaining crystal form I via suspending-slurry washing, volatilizing, cooling, or the like;
placing the compound of formula (1) in a solvent such as anhydrous lower alcohols or tetrahydrofuran, heating the mixture until dissolved, cooling to obtain crystal form II;
placing the compound of formula (1) in a lower alcohol, heating the mixture until dissolved, adding water thereto, cooling to 20°C or less, and filtering to obtain crystal form III; or
heating crystal form I of the compound of formula (1) to 100°C or higher to obtain metastable crystal form V; placing crystal form V at ambient temperature and humidity to transform into crystal form IV.

7. A pharmaceutical composition comprising the crystal form of the compound of formula (1) according to any one of claims 1-5 and a pharmaceutically acceptable carrier, wherein said crystal form is selected from crystal forms I, II, III, and IV, and combinations thereof.

8. A pharmaceutical formulation comprising the crystal form of the compound of formula (1) according to any one of claims 1-5 and a pharmaceutically acceptable carrier and/or diluent, wherein said crystal form is selected from crystal forms I, II, III, and IV, and combinations thereof.

9. The pharmaceutical formulation according to claim 8, **characterized in that** said pharmaceutical formulation is an oral formulation.

10. Use of the crystal form of the compound of formula (1) according to any one of claims 1-5 in manufacture of medicament for lowering uric acid or reducing inflammation, wherein said crystal form is selected from crystal forms I, II, III, and IV, and combinations thereof.

11. Use of the crystal form of the compound of formula (1) according to any one of claims 1-5 in manufacture of medicament for treating and/or preventing uratic and/or gouty diseases, wherein said crystal form is selected from crystal forms I, II, III, and IV, and combinations thereof.

12. The crystal form of the compound of formula (1) according to any one of claims 1-5 for use in lowering uric acid or reducing inflammation, wherein said crystal form is selected from crystal forms I, II, III, and IV, and combinations thereof.

13. The crystal form of the compound of formula (1) according to any one of claims 1-5 for use in treating and/or preventing uratic and/or gouty diseases, wherein said crystal form is selected from crystal forms I, II, III, and IV, and combinations thereof.

14. A method for lowering uric acid or reducing inflammation, which comprises administering the crystal form of the compound of formula (1) according to any one of claims 1-5 to a subject in need thereof, wherein said crystal form is selected from crystal forms I, II, III, and IV, and combinations thereof.

15. A method for treating and/or preventing uratic and/or gouty diseases, which comprises administering the crystal form of the compound of formula (1) according to any one of claims 1-5 to a subject in need thereof, wherein said crystal form is selected from crystal forms I, II, III, and IV, and combinations thereof.
